# FASCICULE DE BREVET EUROPEEN

(11) **EP 1 909 862 B1**
(45) Date de publication et mention de la délivrance du brevet: **14.01.2009**
(21) Numéro de dépôt: 06794242.5
(22) Date de dépôt: 28.07.2006
(51) Int. Cl.: A61L 31/14, A61F 2/00

(54) **TISSU PROTHÉTÎQUE TRIDIMENSIONNEL À FACE DENSE RÉSORBABLE**
DREIDIMENSIONALES PROTHESENGEWEBE MIT EINER RESORBIERBAREN DICHTEN DECKSCHICHT
THREE-DIMENSIONAL PROSTHETIC TISSUE PROVIDED WITH A RESORBABLE DENSE FACE

(30) Priorité: 16.03.2006 FR 0602316; 03.08.2005 US 704869 P
(43) Date de publication de la demande: 16.04.2008
(73) Titulaire: SOFRADIM PRODUCTION, 01600 Trévoux (FR)
(72) Inventeur: THERIN, Michel, F-69004 Lyon (FR); MENEGHIN, Alfredo, F-69009 Lyon (FR); TAYOT, Jean-Louis, F-69890 La Tour de Salvagny (FR); MONTANARI, Suzelei, F-01600 Trevoux (FR)
(74) Mandataire: Brédeville, Odile Marie
(86) Numéro de dépôt international: PCT/FR2006/001848
(87) Numéro de publication internationale: WO 2007/014995

(56) Documents cités:
- EP-A- 1 052 319
- WO-A-99/51163
- WO-A-20/05018698
- DE-A1- 19 912 648
- FR-A- 2 257 262
- FR-A1- 2 766 698
- US-A- 5 569 273
- US-A1- 2002 116 070
- US-A1- 2004 054 406

## Description

La présente invention porte sur un tissu prothétique tridimensionnel, utile notamment pour l'obtention de prothèses de renfort en chirurgie pariétale et/ou viscérale, et particulièrement adapté à la prévention des adhérences post-chirurgicales en cas d'utilisation par voie intra-péritonéale.

Les adhérences post-chirurgicales comprennent toutes les liaisons fibreuses non anatomiques, induites fortuitement par un acte chirurgical lors du processus normal de cicatrisation. Elles peuvent survenir dans toutes les disciplines chirurgicales quel que soit le geste considéré. Elles sont généralement d'autant plus sévères que le traumatisme chirurgical est important et que les tissus assurant normalement les plans de clivage (tissu conjonctif interstitiel, les synoviales, les gaines tendineuses, séreuses péritonéale et pleurale...) ont été touchés. Tout traumatisme chirurgical tissulaire est suivi d'une cascade d'évènements physiologiques dont les principaux temps peuvent être simplifiés comme suit :
- temps zéro (t0) : traumatisme chirurgical, effraction capillaire ;
- temps zéro plus quelques minutes : coagulation, formation du réseau fibrineux, libération des facteurs chimiotactiques ;
- temps zéro (t0) plus 12 à 48 heures : afflux leucocytaire à dominante polynucléaire ;
- temps zéro (t0) plus 24 heures à 5 jours : afflux leucocytaire à dominante macrophagique ;
- temps zéro (t0) plus 4 à 8 jours : afflux fibroblastique ;
- temps zéro (t0) plus 5 à 14 jours : différenciation conjonctive de la réaction cicatricielle ;
- temps zéro (t0) plus 15 à 180 jours : remodelage cicatriciel.

Même si les mécanismes exacts sont pour certains encore inconnus, notamment en ce qui concerne le déterminisme de l'intensité de la réaction, il apparaît donc que les premiers jours sont déterminants puisqu'ils conditionnent l'afflux fibroblastique responsable de la formation d'adhérences.

De ce fait, de telles adhérences post-chirurgicales peuvent provoquer des syndromes pouvant se classer principalement en douleurs chroniques, syndromes occlusifs, et infertilité féminine. Par ailleurs, elles augmentent très sensiblement les risques de fausses routes lors d'une réintervention, (effraction myocardique ou intestinale lors de la thoracotomie ou laparotomie itérative), tout en prolongeant les temps opératoires, la dissection préalable pouvant être dans de tels cas très fastidieuse.

Par ailleurs, en chirurgie viscérale et pariétale, la prothèse de renfort doit présenter une certaine résistance mécanique lui permettant de remplir sa fonction en tant qu'élément de reconstruction chirurgicale. De manière générale, les tissus prothétiques connus notamment dans le traitement des insuffisances pariétales, par exemple hernies et éventrations, apportent un complément de résistance mécanique à la reconstruction chirurgicale. De tels tissus sont d'autant plus efficaces et leur tolérance locale d'autant meilleure que leur intégration tissulaire est intime et précoce. Pour cette raison, des tissus prothétiques particulièrement performants dans ces indications sont des tissus tridimensionnels qui présentent une porosité importante, et sont conçus de façon à être intégrés dans le corps le plus rapidement possible.

De tels tissus prothétiques tridimensionnels ajourés sont par exemple décrits dans WO99/05990.

Pour tenter de pallier le problème des adhérences chirurgicales viscérales consécutives à une intervention d'implantation d'une telle prothèse de renfort en site intra-péritonéal, on a proposé d'interposer une barrière physique entre ledit tissu prothétique tridimensionnel de la prothèse de renfort et les structures organiques adjacentes que l'on souhaite ne pas voir adhérer. L'effet barrière recherché pose toutefois le problème du pouvoir adhésiogène intrinsèque de cette barrière. En effet, si la barrière est constituée d'un matériau non résorbable, elle peut être à l'origine elle-même d'adhérences au cours du temps ; et si elle est résorbable, sa résorption doit être suffisamment peu inflammatoire pour ne pas engendrer elle-même des adhérences.

En particulier, pour éviter ce dernier phénomène, on a proposé de recouvrir une face de la prothèse de renfort par un matériau lisse et non poreux de façon à ne pas offrir d'espace à une recolonisation cellulaire. Ainsi, dans WO99/06079 et WO99/06080, on a proposé un tissu prothétique tridimensionnel ajouré recouvert sur l'une de ses faces d'un film résorbable lisse et non poreux soit à base de polysaccharides, soit à base de collagène.

Toutefois, la fabrication de telles prothèses demande l'association de deux étapes différentes, à la suite l'une de l'autre, à savoir, dans un premier temps, la réalisation de la structure textile tridimensionnelle puis, dans un deuxième temps, le traitement d'une face de la prothèse textile afin de la rendre lisse et non poreuse. Une méthode de traitement de cette face consiste à tremper la prothèse textile dans un hydrogel pour en imprégner ladite face. L'ensemble est ensuite soumis à une opération de séchage, pour que l'hydrogel se transforme en un film sec continu lisse et non poreux.

Alternativement, on peut réaliser une prothèse composite, associant une face textile à une face polymérique anti-adhérences, résorbable ou non résorbable. Dans ces prothèses composites, les deux faces, respectivement textile et polymérique, sont collées l'une à l'autre.

Quelle que soit la méthode de traitement utilisée, cette dernière présente des désavantages : longue et fastidieuse dans le premier cas, peu performante dans le deuxième cas du fait de la fragilité et de l'instabilité de la liaison face textile-face polymérique dans le cas de la prothèse composite.

Ainsi, il existe le besoin d'une prothèse de renfort simple et facile à fabriquer, fiable, qui présenterait d'une part les propriétés mécaniques suffisantes pour assurer son rôle de renforcement d'une paroi tissulaire mais qui d'autre part limiterait, voire empêcherait le développement des adhérences post-chirurgicales sur la face au contact des viscères.

La présente invention vise à remédier à ce besoin en proposant un tissu tridimensionnel, en particulier fait d'une seule pièce, présentant une face poreuse pour favoriser la colonisation cellulaire, et une face dense résorbable.

La présente invention porte sur un tissu prothétique tridimensionnel comprenant une première et une deuxième faces, ladite première face et ladite deuxième face étant opposées et séparées l'une de l'autre par l'épaisseur dudit tissu, ladite première face étant poreuse, caractérisé en ce que ladite deuxième face est dense et réalisée en au moins un premier fil, résorbable.

Le tissu selon l'invention est particulièrement adapté à une utilisation en tant que prothèse de renfort en chirurgie pariétale et viscérale.

La présente invention porte encore sur une prothèse pour renforcer, protéger ou soutenir une paroi tissulaire, caractérisée en ce qu'elle est obtenue par découpage à partir d'un tissu prothétique tel que décrit ci-dessus.

Le tissu et/ou la prothèse selon l'invention permettent une intégration tissulaire la plus rapide possible de la face au contact de la paroi à renforcer, procurant un ancrage mécaniquement satisfaisant, sans fibrose extensive source d'inconfort ou de douleur, tout en empêchant la formation d'adhérences post-chirurgicales au contact des organes environnant la face viscérale ou intra-péritonéale.

Par ailleurs, du fait du caractère résorbable de la face dense du tissu et/ou prothèse selon l'invention, la quantité de matériau non résorbable est réduite et toute réaction inflammatoire chronique potentielle au contact dudit matériau non résorbable est ainsi limitée. Ainsi, la prothèse selon l'invention possède un pouvoir inflammatoire intrinsèque long terme considérablement diminué par rapport à toute autre prothèse classique ne comportant pas une face dense résorbable.

Dans la présente demande, on entend, par « tissu », un assemblage ou arrangement de fils, monofilament ou multifilaments, obtenu par tricotage et/ou tissage.

Dans la présente demande, par « tissu prothétique », on entend un tissu destiné à être implanté dans le corps humain ou animal sous la forme d'une prothèse ou de toute autre pièce façonnée au moins en partie avec ledit tissu.

Dans la présente demande, on entend par « tissu tridimensionnel » un tissu présentant une épaisseur significative, de préférence supérieure ou égale à 0,5 mm.

Dans la présente demande, on entend par « face poreuse » une face dont la surface présente une certaine rugosité, par exemple des alvéoles, trous ou orifices, débouchant dans sa surface, répartis régulièrement ou non, favorisant toute colonisation cellulaire. La face poreuse est la face du tissu prothétique destinée à être au contact avec, puis à s'intégrer à, la paroi tissulaire à renforcer ou à protéger.

Dans la présente demande, on entend par « face dense » une face présentant par endroits des espaces poreux mais dont la surface globale présente une certaine unité et une homogénéité générales. Cette face dense est destinée à être exposée aux viscères adjacents à la paroi tissulaire, pendant la réparation ou régénération de cette dernière.

Dans la présente demande, on entend par « résorbable » la caractéristique selon laquelle un matériau est absorbé par les tissus biologiques et disparaît in vivo au bout d'une période donnée, par exemple en moins de 3 mois, ou encore en moins 4 semaines, ou encore en moins de quelques jours.

Dans la présente demande, on entend, par « entretoise », la ou les nappes qui relie(nt) les deux faces d'un tissu tridimensionnel l'une à l'autre, constituant ainsi l'épaisseur d'un tel tissu.

Selon la présente invention, la face dense est réalisée en un premier fil résorbable. Ce ou ces fils résorbables sont aptes à se transformer partiellement ou totalement in vivo, au contact des tissus organiques et de leurs sécrétions, en un hydrogel continu capable d'assurer la fonction de prévention des adhérences.

De préférence, ledit premier fil résorbable est constitué de monofilaments et/ou multifilaments d'un matériau polymère biocompatible choisi parmi les polyesters, les polycaprolactones, les polydioxanones, les polyamides, les polyéthers, les polysaccharides et leurs mélanges.

Les polyesters peuvent être choisis parmi les polyhydroxyacides, de préférence les polymères d'acide glycolique, les polymères d'acide lactique, les polymères d'acide hydroxybutyrique et leurs mélanges.

Les polysaccharides peuvent être choisis parmi l'acide hyaluronique, l'acide alginique, l'acide polyglucuronique, le chitosan, l'amidon, les dérivés de cellulose solubles, leurs sels et leurs mélanges. Ledit polysaccharide peut être réticulé.

L'acide polyglucuronique peut être d'origine bactérienne, comme le polysaccharide sécrété par la souche mutante de Rhizobium meliloti M5N1CS(NCIMB 40472), selon l'enseignement du brevet WO9318174, ou encore il peut être obtenu par oxydation sélective des hydroxyles primaires de la cellulose.

Les dérivés de cellulose solubles peuvent être choisis parmi les éthers de cellulose, comme la carboxyméthylcellulose, les celluloses oxydées et leurs mélanges.

De préférence, les celluloses oxydées sont choisies parmi la cellulose oxydée où l'alcool primaire en C₆ est partiellement ou totalement oxydé en acide carboxylique, par exemple pour donner de l'acide polyglucuronique, la cellulose oxydée sous forme de polyaldéhydes par l'acide périodique, la cellulose de type « viscose », fabriquée à partir d'une pâte de cellulose solubilisée puis régénérée et oxydée, et leurs mélanges.

Plusieurs variétés de cellulose régénérée ont été développées industriellement. On peut citer par exemple le procédé « viscose » qui est basé sur la solubilité du xanthate de cellulose dans une solution diluée d'hydroxyde de sodium. On peut aussi citer le procédé dit « procédé cupro-ammonium » mis en oeuvre par exemple par la société Bemberg en Italie ou la société Asahi Chemical Industries au Japon, et qui consiste à dissoudre la cellulose dans une solution ammoniacale de cuivre. Un autre procédé de préparation de cellulose régénérée convenant à la présente invention est le procédé de dissolution de cellulose en phase organique par l'oxyde de N-méthylmorpholine (N.M.M.O.), dit « procédé Lyocell®», mis en oeuvre par exemple par la société Lenzing en Autriche.

Filée à travers une plaque perforée, la viscose coagule en milieu acide et forme de longs filaments continus de cellulose régénérée, qui sont séchés et réunis en fils multifilaments. On obtient un fil de cellulose régénérée présentant une bonne résistance mécanique.

De façon générale, un tel fil de cellulose régénérée n'est pas résorbable. Ainsi, comme il sera décrit plus loin dans la présente demande, de préférence, on réalisera dans un premier temps la face dense du tissu selon l'invention avec un tel fil de cellulose régénérée puis dans un deuxième temps, on soumettra cette face dense à un procédé d'oxydation afin de rendre ledit fil de cellulose régénérée résorbable.

A titre d'exemple, on peut citer comme fil de cellulose régénérée convenant à la présente invention le fil multifilaments 90 decitex commercialisé sous le nom « CUPRO® Cusio » par la société italienne Bemberg.

Dans une forme de réalisation de l'invention, ledit premier fil résorbable est un fil multifilaments de cellulose régénérée et oxydée.

Dans une autre forme de réalisation de l'invention, ledit premier fil résorbable est un fil multifilaments composite d'acide polyglycolique et de cellulose oxydée sous forme d'acide polyglucuronique.

Dans une autre forme de réalisation de l'invention, ledit premier fil résorbable est un fil de chitosan, ou un fil d'acide hyaluronique réticulé. Un tel fil d'acide hyaluronique réticulé est à base d'un polymère réalisé avec un faible degré de réticulation tel qu'il puisse s'hydrater rapidement et se dégrader en moins de quatre semaines.

Dans encore une autre forme de réalisation de l'invention, ledit premier fil résorbable est obtenu par mélange d'un polysaccharide de charge négative, choisi parmi l'acide alginique, l'acide hyaluronique, l'acide polyglucuronique et leurs mélanges, et d'un polysaccharide de charge positive, comme le chitosan.

Selon une forme de réalisation de l'invention, ledit premier fil résorbable est un fil multifilaments composé de multiples filaments définissant entre eux des espaces intersticiels, et ledit fil multifilaments est imprégné, au niveau desdits espaces intersticiels, d'un polysaccharide choisi parmi l'acide hyaluronique, l'acide alginique, l'acide polyglucuronique, le chitosan, l'amidon, les dérivés de cellulose solubles et leurs mélanges.

L'imprégnation par une solution visqueuse de polysaccharides est de préférence réalisée par passage des fils secs dans un bain de la solution ou plusieurs bains successifs de solutions différentes. En sortie, les fils peuvent être séchés directement avant d'être bobinés. La phase de séchage peut être précédée d'une phase de coagulation des polysaccharides dans un solvant volatile de type acétone ou isopropanol. Ce solvant peut apporter en même temps un agent de réticulation des chaînes de polysaccharides tel qu'un réactif bifonctionnel de type biépoxyde, comme le butane-diol-diglycidyl-ether, qui réagira avec le polysaccharide à chaud pendant la phase de séchage. Dans un mode particulier de réalisation de l'invention, la réticulation des polysaccharides est obtenue par des liaisons électrostatiques naturelles entre les charges opposées de deux polysaccharides mélangés de charge opposée et de préférence par dépôts successifs d'une première couche de chitosan de charge positive, puis d'une deuxième couche d'un polysaccharide de charge négative, comme par exemple l'acide hyaluronique.

Grâce à cette imprégnation le fil devient très hydrophile en surface et le polysaccharide choisi sera libéré ou hydraté rapidement en présence de l'humidité des tissus biologiques, ou grâce à un mouillage du tissu prothétique selon l'invention avant implantation chez le patient, créant un gel visqueux ancré dans les mailles du tissu, grâce à sa viscosité et sa réticulation éventuelle. La création spontanée de ce gel continu confère au tissu selon l'invention des propriétés de prévention des adhérences.

La nature résorbable de la face dense du tissu selon l'invention, en particulier lorsque les fils résorbables constituant cette face dense sont imprégnés de polysaccharides comme décrit ci-dessus, permet de transformer une face textile discontinue, par exemple réalisée par tricotage, en une face gélifiée continue, par un mouillage préalable à l'implantation ou par la simple mise au contact des tissus biologiques à réparer et à protéger.

Ainsi, ledit premier fil résorbable peut être un fil multifilaments d'acide polyglycolique imprégné d'acide polyglucuronique.

Dans une autre forme de réalisation de l'invention, ledit premier fil résorbable est un fil multifilaments d'acide polyglycolique imprégné d'un mélange d'acide polyglucuronique et de chitosan.

Dans une autre forme de réalisation de l'invention, ledit premier fil résorbable est un fil multifilaments d'acide polyglycolique imprégné d'un mélange d'acide hyaluronique et de chitosan.

Dans une autre forme de réalisation de l'invention, ledit premier fil résorbable est un fil multifilaments d'acide polyglycolique imprégné de hyaluronate réticulé par le 1-4-butane-diol-diglycidyl-éther.

Dans une autre forme de réalisation de l'invention, ledit premier fil résorbable est un fil multifilaments de cellulose oxydée imprégné de chitosan.

Selon une forme de réalisation de l'invention, ladite première face, poreuse, est réalisée en au moins un deuxième fil, résorbable ou non, constitué de monofilaments et/ou multifilaments d'un matériau polymère biocompatible.

De préférence, ledit deuxième fil est non résorbable. La fonction de renfort mécanique du tissu est ainsi garantie de manière définitive et les risques de récidives de hernies par résorption de l'implant sont évitées.

Selon une forme de réalisation de l'invention, ledit deuxième fil est constitué de monofilaments et/ou multifilaments d'un matériau polymère biocompatible choisi parmi le polypropylène, le polyéthylène-téréphtalate le polytetrafluoroethylene, le polyamide, le polyvinyldifluorene et leurs mélanges.

Dans une autre forme de réalisation, la face poreuse est réalisée en au moins un deuxième fil résorbable, par exemple en acide polylactique. Ce cas est particulièrement adapté lorsque les risques de récidive sont faibles ou encore lorsque la qualité de la régénération tissulaire peut être optimale.

Selon une forme de réalisation de l'invention, ladite première face et ladite deuxième face sont reliées l'une à l'autre par une entretoise réalisée en au moins un troisième fil résorbable ou non, constitué de monofilaments et/ou multifilaments d'un matériau polymère biocompatible.

De préférence, ledit troisième fil est résorbable. Ce cas est particulièrement préféré lorsque la fonction de renfort peut être remplie par une seule face poreuse non résorbable.

Ainsi, de préférence, ledit troisième fil est résorbable et est constitué de monofilaments et/ou multifilaments d'un matériau polymère biocompatible choisi parmi les polyesters, les polycaprolactones, les polydioxanones, les polyalkanoates, les polyamides, les polyphosphazènes, les polyacétals, les polyuréthanes, les polyorthoesters, les polycarbonates, les polyanhydrides et leurs mélanges.

Les polyesters peuvent être choisis parmi les polyhydroxyacides, de préférence parmi les polymères d'acide glycolique, les polymères d'acide lactique, les polymères d'acide hydroxybutyrique et leurs mélanges.

Dans une forme de réalisation de l'invention, ledit troisième fil est un fil multifilaments en acide polylactique.

Dans une autre forme de réalisation de l'invention, ledit troisième fil est un fil multifilaments en acide polyglycolique.

Dans encore une autre forme de réalisation de l'invention, ledit troisième fil est un fil multifilaments en acide polylactique et glycolique.

Dans une autre forme de réalisation ledit troisième fil est non résorbable. C'est par exemple le cas pour des surfaces et/ou des volumes de paroi à réparer très importantes ou soumises à des tensions anatomiques supérieures à la moyenne. La paroi régénérée est ainsi renforcée de façon permamente par un tissu non résorbable plus épais que la seule face poreuse.

Selon une forme de réalisation de l'invention, ladite face dense comprend au moins une nappe de premiers fils, tricotés serrés et déterminant une face unie mais perméable. Le tricotage serré des fils résorbables de la face dense permet l'obtention d'un hydrogel continu, par imprégnation aqueuse du tissu ou de la prothèse selon l'invention préalablement à l'implantation, ou lors du contact de ces fils avec l'humidité naturelle des tissus organiques, une fois que le tissu ou la prothèse selon l'invention est implanté(e).

Selon une forme de réalisation de l'invention, ladite face poreuse comprend au moins deux nappes de deuxièmes fils, tricotés; déterminant des ouvertures de forme hexagonale.

Dans une forme de réalisation de l'invention, ladite entretoise comprend au moins une nappe de troisièmes fils, s'étendant sensiblement perpendiculairement depuis la face poreuse vers la faces dense, lesdits troisièmes fils étant distribués, au niveau de ladite face poreuse, selon les bords périphériques desdites ouvertures hexagonales.

De préférence, lesdits troisièmes fils forment des canaux transversaux, sensiblement parallèles les uns aux autres, dont la section interne est exempte de fils, lesdits canaux débouchant de part et d'autre du tissu, sur la face poreuse selon lesdites ouvertures hexagonales, et sur la face dense, respectivement.

De préférence, le diamètre moyen des canaux transversaux est égal ou supérieur à 0,3 mm, de préférence allant de 0,7 à 3 mm, et de préférence encore allant de 1,3 à 1,7 mm.

De préférence, les canaux transversaux présentent une longueur, correspondant à l'épaisseur dudit tissu, allant de 0,5 à 5 mm, de préférence encore allant de 1,5 à 3 mm.

De préférence, l'entretoise détermine pour chaque canal une paroi interne poreuse d'interconnexion avec les canaux voisins, ladite paroi interne poreuse définissant des interstices de passage entre canaux.

Avantageusement, lesdits interstices de passage entre canaux ont une largeur allant de 100 à 300 microns.

La porosité de la paroi des canaux est déterminée en particulier par l'agencement textile desdits troisièmes fils qui peuvent présenter à titre d'exemple un diamètre compris entre 10 et 15 µm.

Les parois des canaux fournissent une zone d'ancrage pour la réaction fibreuse sous dépendance de la prothèse (environs immédiats de chaque fil), ce qui contribue néanmoins à une intégration tissulaire du tissu prothétique relativement intime et précoce. De surcroît, lorsque la section interne de chaque alvéole ou canal est substantiellement exempte de tout fil de liaison, on diminue d'autant la réaction inflammatoire du tissu prothétique in vivo, limitant ainsi la formation de capsule fibreuse périphérique responsable de contraction cicatricielle secondaire. Cette structure tridimensionnelle très poreuse du tissu selon l'invention permet une différenciation d'un tissu conjonctif histologiquement normal au coeur de la prothèse. La porosité multidirectionnelle favorise en outre le drainage du site et limite ainsi les risques associés aux collections de liquides (séromes, hématomes, sepsis).

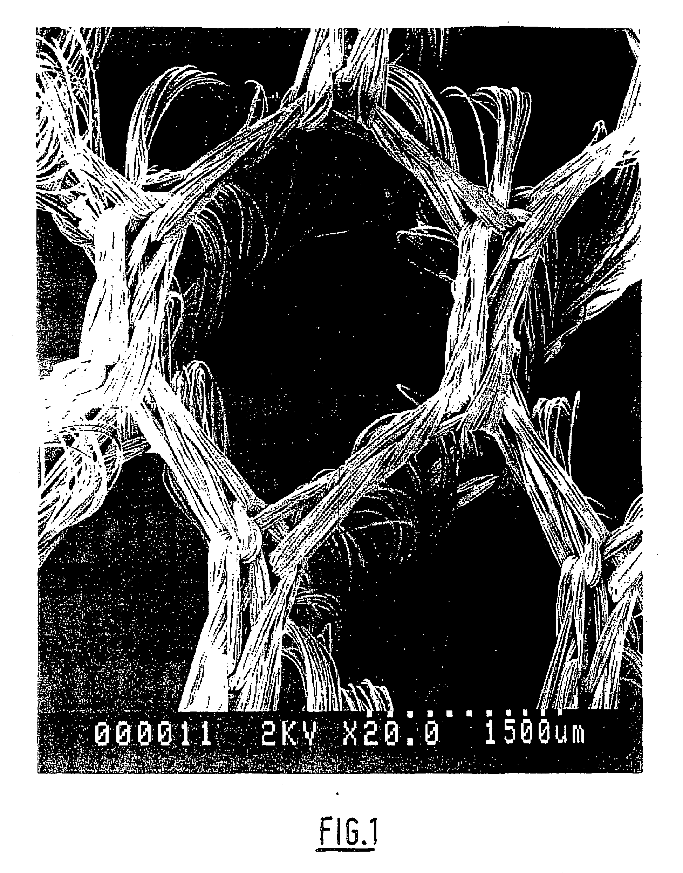

Une fois le tissu prothétique implanté, les cellules, présentes au centre du volume créé par la structure tridimensionnelle, se trouvent à au moins 750 µm de tout matériel prothétique, si les conditions dimensionnelles définies ci-dessus sont respectées et comme montré plus loin sur la figure 1 accompagnant la présente description. Ainsi, les cellules colonisatrices sont loin de toute influence pouvant retarder ou perturber les mécanismes de différenciation, tout en étant à moins de un millimètre du tissu receveur, c'est-à-dire proches des éléments fournissant les éléments indispensables à une réhabitation rapide (cellules souches progénitrices, capillaires sanguins, etc...).

Ces conditions permettent d'obtenir un ancrage mécaniquement satisfaisant tout en préservant une différenciation achevée à coeur, telle que rencontrée dans un tissu conjonctif normal. Lorsque cette entretoise est constituée de fils résorbables, toute réaction inflammatoire aura disparu après résorption de ces fils. Le tissu conjonctif recréé reste stable, pour autant qu'il a pu se développer et se différencier dans l'architecture poreuse des prothèses selon l'invention.

Grâce à l'architecture de l'espace créé par le tissu et/ou la prothèse tridimensionnelle selon l'invention, et notamment la dimension des pores et leurs interconnexions, le tissu et/ou la prothèse selon l'invention permettent une colonisation cellulaire et une intégration tissulaire optimales.

De plus, une fois que le tissu ou la prothèse selon l'invention est implanté(e), un tissu de régénération se développe progressivement au niveau de la face dense, au contact du tissu régénéré dans la face poreuse et l'entretoise. Ce tissu de régénération recrée un feuillet tissulaire de couverture, bien structuré et stable, même après dégradation de la face dense résorbable. Ce feuillet tissulaire met définitivement tout organe adjacent à distance de la partie non résorbable du tissu ou de la prothèse limitant ainsi les risques d'adhérences viscérales post-chirurgicales.

Par ailleurs, la face dense du tissu ou prothèse selon l'invention faisant partie intégrante dudit tissu ou de ladite prothèse, avant implantation, cette face dense est parfaitement stable et ne risque pas de se séparer ou de se délaminer dudit tissu ou de ladite prothèse, comme par exemple dans le cas des prothèses composites réalisées en deux temps par exemple par collage.

Dans les tissus et prothèses selon l'invention, lesdits premiers, deuxièmes et troisièmes fils peuvent être identiques ou différents. En particulier, le tissu et la prothèse selon l'invention peuvent être entièrement résorbables, par exemple dans le cas où la fonction de renfort de paroi tissulaire de la prothèse n'est souhaitée que pour une période provisoire.

En général toutefois, la fonction de renfort de la prothèse sera souhaitée de façon permanente et lesdits deuxièmes fils, constitutifs de la face poreuse, seront différents desdits premiers fils constitutifs de la face dense, et seront non résorbables. Dans ce cas, lesdits troisièmes fils, constitutifs de l'entretoise, pourront être non résorbables, identiques ou non auxdits deuxièmes fils, ou bien résorbables, identiques ou non auxdits premiers fils constitutifs de la face dense.

Enfin, les tissus et prothèses selon l'invention sont particulièrement simples et rapides à fabriquer. En effet, selon un premier mode de réalisation de l'invention, le procédé de préparation des tissus et prothèses de l'invention peut être réalisé en une seule étape, par exemple par tricotage ou encore par tissage, et ce procédé ne nécessite pas alors d'opération spécifique de traitement d'une face du tissu pour rendre la face dense résorbable. Ceci est particulièrement vrai lorsqu'on peut choisir, avant tricotage ou tissage, un fil simple ou composite de nature résorbable en vue de constituer la face dense.

Selon un deuxième mode de réalisation de l'invention, le procédé de préparation des tissus et prothèses de l'invention comprend une première étape de fabrication du tissu, par exemple tricotage ou tissage, puis une étape ultérieure d'oxydation dudit tissu. Dans un tel cas, il est possible de choisir, comme fil destiné à servir de fil constitutif de la face dense un fil non résorbable avant oxydation, et résorbable après oxydation. C'est par exemple le cas lorsqu'on choisit, comme fil destiné à servir de fil constitutif de la face dense, un fil en cellulose régénérée, par exemple non oxydée. Le fil en cellulose régénérée non oxydée devient résorbable après une étape d'oxydation.

Dans tous les cas, la formation d'un film d'hydrogel continu, au niveau de la face dense du tissu et des prothèses selon l'invention, comme vu plus haut, ne nécessite pas d'étape particulière de fabrication : ce film se forme par simple mouillage du tissu ou de la prothèse selon l'invention avant implantation, ou lors du contact du tissu ou de la prothèse selon l'invention avec les sécrétions aqueuses des tissus organiques à protéger.

Un autre objet de l'invention porte sur un procédé de préparation d'un tissu prothétique tridimensionnel comprenant une première et une deuxième faces, ladite première face et ladite deuxième face étant opposées et séparées l'une de l'autre par l'épaisseur dudit tissu, ladite première face étant poreuse, ladite deuxième face étant dense et résorbable, comprenant une étape de fabrication d'un tricot tridimensionnel sur un métier chaîne ou Rachel selon au moins une nappe de fils définissant la face poreuse, au moins une nappe de fils définissant l'épaisseur dudit tissu, et au moins une nappe de fil définissant ladite face dense, caractérisé en ce que ladite nappe définissant ladite face dense est obtenue à l'aide d'une barre à passettes enfilée pleine avec au moins un premier fil, résorbable.

Le tissu peut ensuite être stabilisé simplement par passage au four à une température comprise entre environ 80°C et 150°C.

Encore un autre objet de l'invention porte sur un procédé de préparation d'un tissu prothétique tridimensionnel comprenant une première et une deuxième faces, ladite première face et ladite deuxième face étant opposées et séparées l'une de l'autre par l'épaisseur dudit tissu, ladite première face étant poreuse, ladite deuxième face étant dense et résorbable, comprenant une étape de fabrication d'un tricot tridimensionnel sur un métier chaîne ou Rachel selon au moins une nappe de fils définissant la face poreuse, au moins une nappe de fils définissant l'épaisseur dudit tissu, et au moins une nappe de fil définissant ladite face dense, caractérisé en ce que,
- dans une première étape, ladite nappe définissant ladite face dense est obtenue à l'aide d'une barre à passettes enfilée pleine avec au moins un premier fil en cellulose régénérée, et
- dans une deuxième étape, ledit tissu est soumis à une étape d'oxydation.

Dans une forme de réalisation de l'invention, ledit tissu est soumis à une oxydation par le metapériodate de sodium ou par l'acide périodique, puis rincé dans une solution aqueuse d'acétone ou d'alcool, et lavé à l'acétone ou à l'alcool purs avant séchage. Par exemple, ledit tissu peut être soumis à une oxidation par l'acide périodique 10 mM, pendant 15 heures à température ambiante.

Dans une autre forme de réalisation de l'invention, ledit tissu est soumis à une oxydation par le dioxyde d'azote (NO₂) dans un solvant non aqueux approprié puis lavé par une solution aqueuse d'isopropanol. Il est possible d'utiliser le NO₂ à l'état gazeux seul sans autre solvant liquide ou gazeux. De préférence, ledit tissu est soumis à une oxydation par le dioxyde d'azote gazeux à une température allant de 20 à 50°C, notamment de 25 à 40°C, par exemple pendant une durée allant de 2 à 48 heures, notamment de 3 à 8 heures. Pour éviter la condensation du dioxyde d'azote il est preferable d'utiliser une concentration en dioxyde d'azote inférieure à 14g/L notamment allant de 6 à 12 g/L. Il est préférable que le rapport massique NO₂/cellulose soit supérieur à 0,9 pour assurer une oxydation suffisante. En variante, il est possible de réaliser l'oxydation par le dioxyde d'azote gazeux en présence d'air ou d'oxygène. En variante, il est possible d'utiliser le NO₂ dans un gaz comme le CO₂ ou l'azote ou dans des solvants liquides chlorés ou perfluorés, comme le tétrachlorure de carbone, les fréons ou leurs produits de substitution, selon l'enseignement du brevet US 3, 364, 200. Il est également possible d'utiliser le NO₂ dans un gaz comme le CO₂ ou l'azote maintenu dans un état dense ou supercritique comme décrit dans la demande WO2006018552.

De préférence, ledit tissu, après une oxydation par le dioxyde d'azote gazeux, est soumis à une étape de lavage par un gaz inerte comme le CO₂ ou le N₂ pour éliminer l'excès en NO₂, suivie d'un lavage par un alcool volatil. L'alcool volatil est par exemple l'isopropanol pur.

De préférence, un agent plastifiant, tel que la glycérine ou le polyéthylène glycol est ajouté à l'étape de lavage.

Dans une forme de réalisation de l'invention, lesdits premiers fils de la nappe définissant la face dense sont tricotés selon le barème 1112/1110//.

Dans une autre forme de réalisation de l'invention, lesdits premiers fils de la nappe définissant la face dense sont tricotés selon le barème 2223/1110//.

Selon une forme de réalisation de l'invention, le tissu est stabilisé par passage au four à une température allant de 80 à 150°C avant oxydation.

Les différentes formes de réalisation et les avantages de la présente invention vont maintenant ressortir des dessins annexés dans lesquels :
- la Figure 1 est une vue au microscope électronique à balayage «HITACHI type S 800», grossissement X20, de la face poreuse d'un tissu selon l'invention,
- la Figure 2 est une vue au microscope électronique à balayage «HITACHI type S 800», grossissement X30, de l'entretoise et de la face dense d'une variante du tissu selon l'invention,
- la Figure 3 est une vue au microscope électronique à balayage «HITACHI type S 800», grossissement X30, de la face dense du tissu de la figure 2,
- la Figure 4 est une vue au microscope électronique à balayage «HITACHI type S 800», grossissement X30, d'une section verticale de la face poreuse, de l'entretoise et de la face dense du tissu de la figure 2,
- les Figures 5a et 5b sont des dessins schématiques de deux variantes d'armures de tricotage pour obtenir l'entretoise d'un tissu selon l'invention,
- les Figures 6 et 7 sont des dessins schématiques de deux variantes d'armures de tricotage pour obtenir la face dense d'un tissu selon les figures 2-4,
- la Figure 8 est un dessin schématique d'une armure de tricotage pour obtenir la face poreuse d'un tissu selon la figure 1.

La figure 1 montre un exemple de face poreuse d'un tissu prothétique tridimensionnel selon l'invention. Selon cette figure, la face poreuse est indépendante et elle présente des ouvertures de forme hexagonale. Les ouvertures de cette face sont définies par des bords périphériques, formés avec les fils constitutifs de cette face. De préférence, ces fils sont non résorbables afin d'assurer la fonction permanente de renfort de paroi tissulaire du tissu ou de la prothèse. Dans le cas où cette fonction de renfort n'est souhaitée que pour un temps déterminé, ces fils peuvent être résorbables. Dans le mode d'exécution représenté sur la figure 1, le fil constitutif de la face poreuse est un fil multifilaments de polyester 50 décitex.

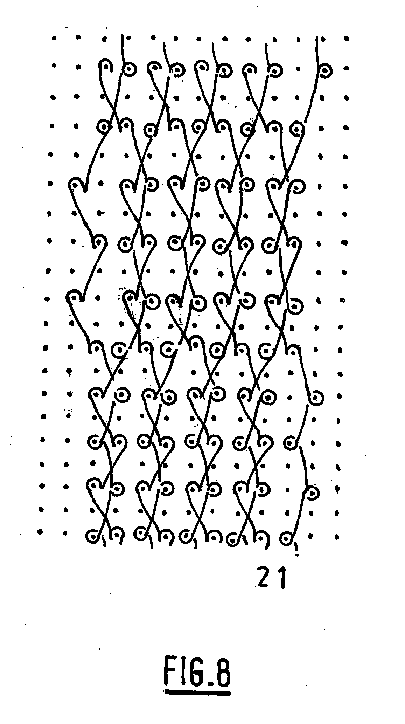

La face poreuse représentée sur la figure 1 est tricotée sur un métier Rachel à double fonture avec deux nappes de fils (1,2), selon l'armure de tricotage représentée sur la figure 8, selon un schéma de représentation classique pour l'homme du métier et qui ne sera pas décrit plus en détails ici. Les barres du métier à tricoter, correspondant aux fils 1 et 2 sont enfilées un plein-un vide. Selon l'armure décrite à la figure 8, les fils sont tricotés selon les barêmes suivants :
- 1211/1011/1211/1011/1222/3222/1222/3222// pour la nappe de fils 1,
- 1222/3222/1222/3222/1211/1011/1211/1011// pour la nappe de fils 2.

Comme il apparaît nettement sur la figure 1, un tel tricotage donne une face poreuse à ouvertures hexagonales de diamètre moyen allant d'environ 1,3 mm à 1,7 mm. Une telle face poreuse est donc tout à fait propice à une bonne colonisation cellulaire et une bonne intégration tissulaire. En effet, comme indiqué, une fois le tissu prothétique implanté, les cellules, présentes au centre du volume créé par la structure tridimensionnelle, se trouvent ainsi à au moins 750 µm de tout matériel prothétique. Ainsi, les cellules colonisatrices sont loin de toute influence pouvant retarder ou perturber les mécanismes de différenciation, tout en étant à moins de un millimètre du tissu biologique receveur, ce qui représente des conditions optimales pour obtenir un ancrage mécaniquement satisfaisant tout en préservant une différenciation achevée à coeur, telle que rencontrée dans un tissu conjonctif normal.

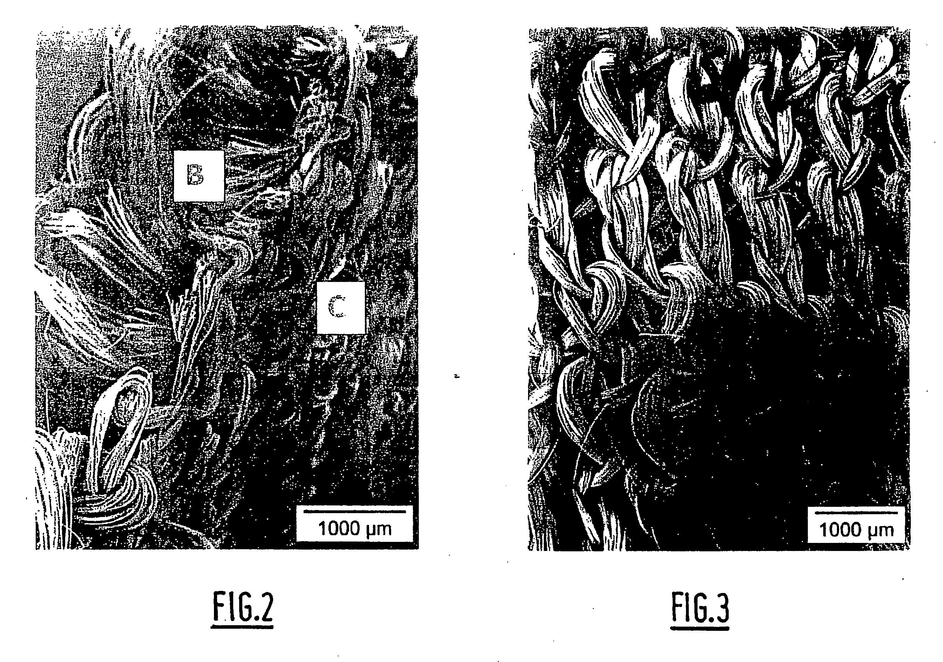

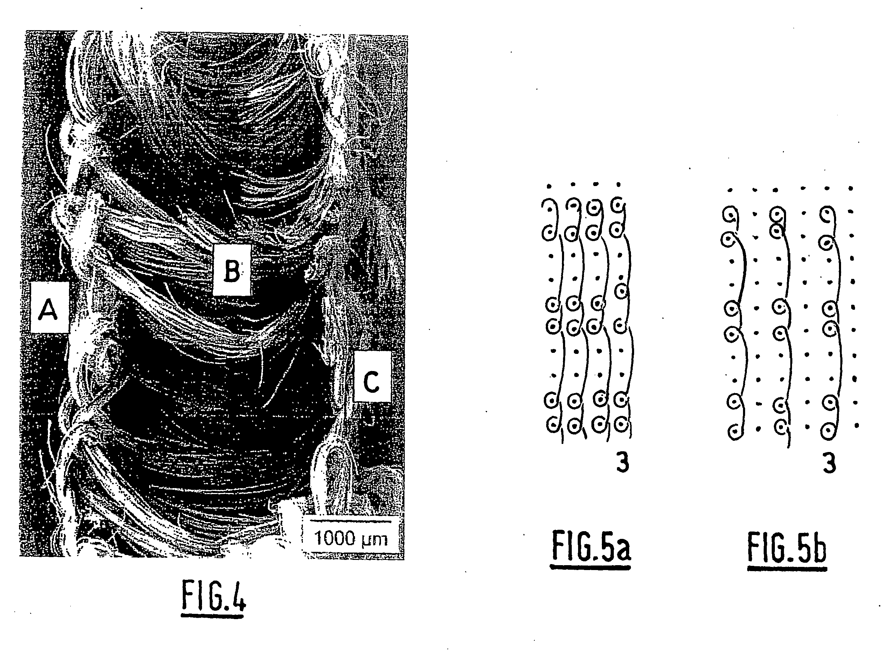

Sur les figures 2-4 est représenté un tissu selon l'invention dont la face poreuse (A) est réalisée selon la même méthode que la face poreuse de la figure 1, mais avec du fil monofilament en polypropylène de diamètre 0,1 mm : un tel fil est disponible commercialement sous le nom « CRINLENE®» auprès de la société italienne SIDER ARC. Comme on peut le voir sur les Figures 2 et 4, la face poreuse (A) et la face dense (C) sont reliées entre elles par l'entretoise (B) qui, sur les figures 2 et 4, comprend une nappe de fils, appelée aussi nappe de liaison intermédiaire, qui s'étend sensiblement perpendiculairement depuis la face poreuse (A) vers la face dense (C). Les fils constitutifs de cette nappe de liaison sont distribués selon les bords périphériques des ouvertures hexagonales de la face poreuse. Les fils de liaison ainsi distribués forment des canaux transversaux, sensiblement parallèles les uns aux autres, dont la section interne est exempte de fils. Ces canaux transversaux débouchent de part et d'autre du tissu, sur la face poreuse et la face dense respectivement.

Conformément à l'invention, les fils de liaison sont disposés en sorte que chaque canal ou alvéole transversal ait une paroi interne poreuse d'interconnexion latérale avec les canaux voisins, ces interstices ayant un diamètre compris entre 100 et 300 µm. Les canaux transversaux augmentent la vitesse de colonisation cellulaire, une fois le tissu implanté in vivo, car ils facilitent l'acheminement ou l'afflux cellulaire au site de l'implantation. Par ailleurs, la quasi-absence de fils dans le volume même des canaux transversaux permet de baisser la réaction inflammatoire du tissu prothétique, ce qui favorise encore une bonne implantation de ce dernier.

L'entretoise représentée sur les figures 2 et 4 est tricotée sur un métier Rachel à double fonture avec une nappe de fils 3, selon l'armure de tricotage représentée sur la figure 5a, selon un schéma de représentation classique pour l'homme du métier. Le fil utilisé est du fil de cellulose régénérée multifilaments 90 decitex, disponible commercialement sous le nom « CUPRO® Cusio » auprès de la société italienne Bemberg. La barre du métier à tricoter, correspondant aux fils 3 est enfilée pleine comme montré sur la figure 5a. Dans une autre forme de réalisation de l'invention, cette barre peut être enfilée un plein-un vide comme représenté sur la figure 5b. Dans encore une autre forme de réalisation de l'invention, cette barre peut être enfilée à disposition irrégulière de pleins et de vides. Selon les armures décrites aux figures 5a et 5b, les fils sont tricotés selon le barème suivant : 0101/0000//.

Sur la figure 3 est représentée la face dense d'un tissu selon l'invention. Comme il apparaît sur cette figure, cette face dense peut être indépendante et elle présente une face unie, dense mais toutefois perméable. Les fils constitutifs de cette face dense sont résorbables et de préférence tricotés serrés comme il apparaît sur la figure 3. Ce tricotage serré et la nature résorbable de ces fils les rendent aptes, lors d'un mouillage préalable à l'implantation ou au contact des tissus organiques in vivo, à transformer cette face dense en hydrogel capable d'assurer la fonction de prévention des adhérences.

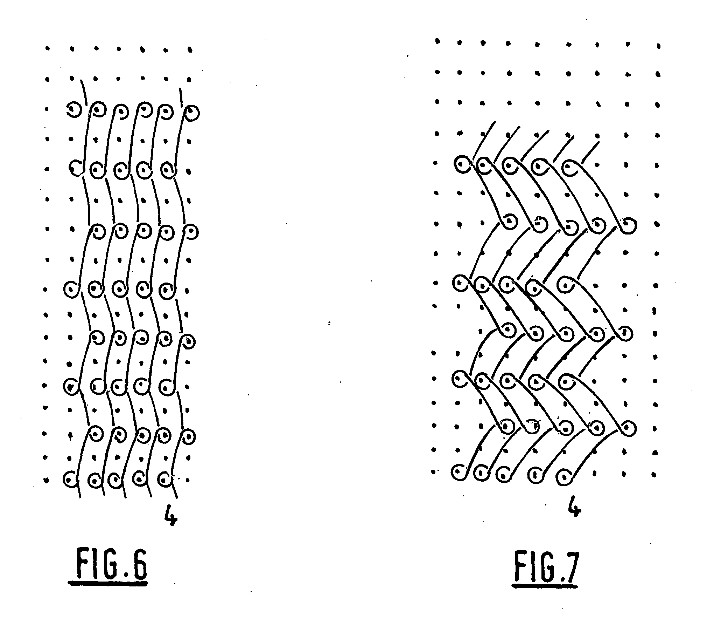

La face dense représentée sur la figure 3 est tricotée sur un métier Rachel à double fonture avec une nappe de fils 4, selon l'armure de tricotage représentée sur la figure 6, selon un schéma de représentation classique pour l'homme du métier. Le fil utilisé pour l'étape de tricotage est le même que pour l'entretoise, à savoir du fil de cellulose régénérée multifilaments 90 decitex, disponible commercialement sous le nom « CUPRO® Cusio » auprès de la société italienne Bemberg. Comme vu plus haut, ce fil n'est pas résorbable avant oxydation. Le tissu est donc tricoté avec ce fil de cellulose régénérée, puis il est soumis à une étape d'oxydation pour oxyder la cellulose et rendre ce fil de cellulose résorbable. Dans le cas du tissu des figures 2 à 4, l'entretoise et la face dense seront donc en fils résorbables après oxydation.

La barre du métier à tricoter, correspondant aux fils 4 est enfilée pleine. Un tel enfilage plein permet une meilleure homogénéité et une bonne densité de la face. Selon l'armure décrite à la figure 6, les fils 4 sont tricotés selon le barème suivant : 1112/1110//.

Dans une autre forme de réalisation de l'invention, la face dense est tricotée selon l'armure représentée sur la figure 7. Dans un tel cas, la barre du métier à tricoter, correspondant aux fils 4 est enfilée pleine et les fils sont tricotés selon le barème suivant : 2223/1110//.

Le tissu tridimensionnel représenté sur la figure 4 peut ainsi être réalisé par tricotage en chaîne des quatre nappes de fils (1, 2, 3, 4) décrites ci-dessus, selon la technique dite de « mailles jetées » sur métier Rachel. De préférence, les différentes nappes 1 à 4 sont toutes tricotées en même temps. Ainsi, les fils de liaison sont distribués selon les bords périphériques des ouvertures de la face poreuse et s'étendent sensiblement perpendiculairement depuis cette face poreuse vers la face dense, en ménageant des interstices d'interconnexion latérale avec les autres canaux, et en évitant que des fils de liaison occupent une partie trop importante du volume des canaux transversaux qui sont formés.

Le tissu peut ensuite être stabilisé simplement par passage au four à une température comprise entre environ 80°C et 150°C. L'épaisseur du tissu obtenu est de l'ordre de 1,5 à 5 mm, et d'un poids d'environ 100 à 250g/m².

Dans une autre forme de réalisation de l'invention, le tissu tridimensionnel est réalisé selon la technique de tricotage dite de « mailles cueillies », ou « maille trame ».

Le tissu tridimensionnel selon l'invention peut aussi être réalisé en tissage, par la technique velours, double nappe, comme par exemple décrit dans l'ouvrage de C. VILLARD « Manuel de théorie du tissage », à la page 229, Lyon, 1948.

La présente invention va maintenant être illustrée par les exemples suivants.

### Exemple 1 :

On réalise sur un métier Rachel, selon la technique décrite aux figures 1 à 8 ci-dessus, un tissu tricoté contenant trois types de fils différents pour respectivement la face poreuse, l'entretoise et la face dense.

La face poreuse est réalisée en un fil non résorbable multifilaments de polyéthylène-téréphtalate. L'entretoise est réalisée en fil multifilaments d'acide polylactique (PLA). La face dense est réalisée en fil multifilaments de cellulose régénérée.

Une fois le tissu tricoté réalisé, il est soumis à une étape d'oxydation par le NO₂.

Cette oxydation est réalisée en faisant agir le NO₂ selon l'enseignement du brevet US 3364200. Le NO₂ est dissous dans un solvant non aqueux de type CO₂ ou N₂ à l'état gazeux, liquide ou supercritique, ou dans un solvant liquide, de type tétrachlorure de carbone, ou fréon 113, ou ses substituts perfluorés. L'oxydation est suivie d'un lavage par le solvant, puis de préférence par un lavage en isopropanol, ou en acétone. Le tissu est ensuite séché sous vide, puis découpé sous formes de prothèses de renfort qui sont emballées et stérilisées à l'oxyde d'éthylène.

Selon ce procédé d'oxydation, seule la cellulose est oxydée. Elle devient progressivement hydrosoluble et résorbable après implantation de la prothèse dans le corps du patient.

Un trempage de la prothèse dans l'eau, juste avant implantation, accélère si nécessaire la transformation de la face dense en hydrogel continu, la présence de cet hydrogel continu étant souhaitable le plus tôt possible pour la prévention d'éventuelles adhérences post-opératoires.

### Exemple 2 :

On réalise un tissu tridimensionnel et des prothèses tricotés selon la méthode décrite dans l'exemple 1 en remplaçant les fils utilisés dans l'exemple 1 par les fils suivants :
- un fil monofilament de polypropylène pour la face poreuse,
- un fil de cellulose régénérée pour l'entretoise et la face dense.

Le tissu ainsi tricoté est ensuite soumis à une étape d'oxydation par le NO₂. Cette oxydation est réalisée en faisant agir le NO₂ gazeux à une concentration de 10 g/L avec une relation de1,3 gramme de NO₂ par gramme de cellulose. La réaction est réalisée pendant 4 heures. A la fin de la réaction on effectue un lavage par un gaz inerte comme le CO₂ ou le N₂ pour éliminer l'excès en NO₂. On procède ensuite à un lavage par le mélange isopropanol/eau (1 :1), puis avec l'isopropanol pur. Le tissu est ensuite séché, puis découpé sous formes de prothèses de renfort qui sont emballées et stérilisées par rayonnement gamma.

### Exemple 3 :

On réalise un tissu tridimensionnel et des prothèses tricotés selon la méthode décrite dans l'exemple 1 en remplaçant les fils utilisés dans l'exemple 1 par les fils suivants :
- un fil monofilament de polypropylène pour la face poreuse,
- un fil multifilaments en acide polyglycolique (PGA) pour l'entretoise,
La face dense est réalisée en fil de cellulose régénérée comme dans l'exemple 1.

Le tissu ainsi tricoté est ensuite soumis à une oxydation comme dans l'exemple 1.

### Exemple 4 :

On réalise sur métier Rachel une prothèse tricotée selon la technique de tricotage décrite aux figures 1 à 8 ci-dessus contenant trois types de fils différents pour respectivement la face poreuse, l'entretoise et la face dense.

La face poreuse est réalisée en un fil non résorbable multifilaments de polyéthylène-téréphtalate. L'entretoise est réalisée en fil multifilaments d'acide polylactique (PLA). La face dense est réalisée en fil composite PGA-Cellulose oxydée sous forme d'acide polyglucuronique.

### Exemple 5 :

On réalise sur métier Rachel une prothèse tricotée selon la technique de tricotage décrite aux figures 1 à 8 ci-dessus contenant trois types de fils différents pour respectivement la face poreuse, l'entretoise et la face dense.

La face poreuse est réalisée en un fil non résorbable multifilaments et/ou monofilament de polypropylène. L'entretoise est réalisée en fil multifilaments d'acide polylactique et glycolique (PLGA). La face dense est réalisée en fil de hyaluronate.

### Exemple 6 :

On réalise sur métier Rachel une prothèse tricotée selon la technique de tricotage décrite aux figures 1 à 8 ci-dessus contenant trois types de fils différents pour respectivement la face poreuse, l'entretoise et la face dense.

La face poreuse est réalisée en un fil non résorbable multifilaments de polyéthylène-téréphtalate. L'entretoise est réalisée en fil multifilaments d'acide polylactique (PLA). La face dense est réalisée en fil multifilaments d'acide polyglycolique, imprégné de hyaluronate et/ou d'acide polyglucuronique.

### Exemple 7 :

On réalise un tissu similaire à celui de l'exemple 6, dans lequel le fil de la face dense est imprégné d'un mélange d'acide hyaluronique et de chitosan.

### Exemple 8 :

On réalise sur métier Rachel une prothèse tricotée selon la technique de tricotage décrite aux figures 1 à 8 ci-dessus contenant trois types de fils différents pour respectivement la face poreuse, l'entretoise et la face dense.

La face poreuse est réalisée en un fil non résorbable monofilament de polypropylène. L'entretoise est réalisée en fil multifilaments d'acide polylactique (PLA). La face dense est réalisée en fil multifilaments d'acide polyglycolique (PGA), imprégné de hyaluronate selon la méthode suivante : le fil multifilaments d'acide polyglycolique est imprégné de hyaluronate à pH 9, puis réticulé à chaud par le 1-4-butane-diol-diglycidyl-ether, avant tricotage, au moment du séchage acétonique du fil composite de PGA.

Dans les exemples 6, 7 et 8, les fils imprégnés constitutifs de la phase dense deviennent très hydrophiles en surface et, pour chacun d'eux, le polysaccharide choisi sera libéré ou hydraté rapidement par un mouillage préalable et/ou en présence de l'humidité des tissus biologiques, créant un gel visqueux ancré dans les mailles du tissu selon l'invention, grâce à sa viscosité et sa réticulation éventuelle. La création spontanée de ce gel continu confère au tissu selon l'invention des propriétés de prévention des adhérences.

## Revendications

1. Tissu prothétique tridimensionnel comprenant une première et une deuxième faces, ladite première face et ladite deuxième face étant opposées et séparées l'une de l'autre par l'épaisseur dudit tissu, ladite première face étant poreuse, **caractérisé en ce que** ladite deuxième face est dense et réalisée en au moins un premier fil, résorbable, ladite première face, poreuse, est réalisée en au moins un deuxième fil, résorbable ou non, constitué de monofilaments et/ou multifilaments d'un matériau polymère biocompatible, ladite p emière face et ladite deuxième face sont reliées l'une à l'autre par une entretoise réalisée en au moins un troisième fil résorbable ou non, constitué de monofilaments et/ou multifilaments d'un matériau polymère biocompatible.

2. Tissu selon la revendication précédente, **caractérisé en ce que** ledit premier fil résorbable est constitué de monofilaments et/ou multifilaments d'un matériau polymère biocompatible choisi parmi les polyesters, les polycaprolactones, les polydioxanones, les polyamides, les polyéthers, les polysaccharides et leurs mélanges.

3. Tissu selon la revendication précédente, **caractérisé en ce que** les polyesters sont choisis parmi les polyhydroxyacides, de préférence les polymères d'acide glycolique, les polymères d'acide lactique, les polymères d'acide hydroxybutyrique et leurs mélanges.

4. Tissu selon la revendication 2, **caractérisé en ce que** les polysaccharides sont choisis parmi l'acide hyaluronique l'acide alginique, l'acide polyglucuronique, le chitosan, l'amidon, les dérivés de cellulose solubles, leurs sels et leurs mélanges.

5. Tissu selon la revendication précédente, **caractérisé en ce que** ledit polysaccharide est réticulé.

6. Tissu selon la revendication 4, **caractérisé en ce que** les dérivés de cellulose solubles sont choisis parmi les éthers de cellulose, comme la carboxyméthylcellulose, les celluloses oxydées et leurs mélanges.

7. Tissu selon la revendication précédente, **caractérisé en ce que** les celluloses oxydées sont choisies parmi la cellulose oxydée où l'alcool primaire en C₆ est partiellement ou totalement oxydé en acide carboxylique, par exemple pour donner de l'acide polyglucuronique, la cellulose oxydée sous forme de polyaldéhydes par l'acide périodique, la cellulose de type « viscose », fabriquée à partir d'une pâte de cellulose solubilisée puis régénérée et oxydée, et leurs mélanges.

8. Tissu selon la revendication 7, **caractérisé en ce que** ledit premier fil résorbable est un fil multifilaments de cellulose régénérée et oxydée.

9. Tissu selon les revendications 3 et 7, **caractérisé en ce que** ledit premier fil résorbable est un fil multifilaments composite d'acide polyglycolique et de cellulose oxydée sous forme d'acide polyglucuronique.

10. Tissu selon la revendication 4, **caractérisé en ce que** ledit premier fil résorbable est obtenu par mélange d'un polysaccharide de charge négative, choisi parmi l'acide alginique, l'acide hyaluronique, l'acide polyglucuronique et leurs mélanges, et d'un polysaccharide de charge positive, comme le chitosan.

11. Tissu selon l'une quelconque des revendications précédentes, **caractérisé en ce que** ledit premier fil résorbable étant un fil multifilaments composé de multiples filaments définissant entre eux des espaces intersticiels, ledit fil multifilaments est imprégné, au niveau desdits espaces intersticiels, d'un po ysaccharide choisi parmi l'acide hyaluronique, l'acide alginique, l'acide polyglucuronique, le chitosan, l'amidon, les dérivés de cellulose solubles et leurs mélanges.

12. Tissu selon la revendication précédente, **caractérisé en ce que** ledit premier fil résorbable est un fil multifilaments d'acide polyglycolique imprégné d'acide polyglucuronique.

13. Tissu selon la revendication 11, **caractérisé en ce que** ledit premier fil résorbable est un fil multifilaments d'acide polyglycolique imprégné d'un melange d'acide polyglucuronique et de chitosan.

14. Tissu selon la revendication 11, **caractérisé en ce que** ledit premier fil résorbable est un fil multifilaments d'acide polyglycolique imprégné d'un mélange d'acide hyaluronique et de chitosan.

15. Tissu selon la revendication 11, **caractérisé en ce que** ledit premier fil résorbable est un fil multifilaments d'acide polyglycolique imprégné de hyaluronate réticulé par le 1-4-butane-diol-diglycidyl-éther.

16. Tissu selon la revendication 11, **caractérisé en ce que** ledit premier fil résorbable est un fil multifilaments de cellulose oxydée imprégné de chitosan.

17. Tissu selon l'une quelconque des revendications précédentes **caractérisé en ce que** ledit deuxième fil est non résorbable.

18. Tissu selon la revendication 17, **caractérisé en ce que** ledit deuxième fil est constitué de monofilaments et/ou multifilaments d'un matériau polymère biocompatible choisi parmi le polypropylène, le polyéthylène-téréphtalate le polytetrafluoroethylene, le polyamide, le polyvinyldifluorene et leurs melanges.

19. Tissu selon l'une quelconque des revendications précédentes, **caractérisé en ce que** ledit troisième fil est résorbable.

20. Tissu selon la revendication précédente, **caractérisé en ce que** ledit troisième fil est constitué de monofilaments et/ou multifilaments d'un matériau polymère biocompatible choisi parmi les polyesters, les polycaprolactones, les polydioxanones, les polyalkanoates, les polyamides, les polyphosphazènes, les polyacéltals, les polyuréthanes, les polyorthoesters, les polycarbonates, les polyanhydrides et leurs mélanges.

21. Tissu selon la revendication précédente, **caractérisé en ce que** les polyesters sont choisis parmi les polyhydroxyacides, de préférence parmi les polymères d'acide glycolique, les polymères d'acide lactique, les polymères d'acide hydroxybutyrique et leurs mélanges.

22. Tissu selon la revendication précédente, **caractérisé en ce que** ledit troisième fil est un fil multifilaments en acide polylactique.

23. Tissu selon la revendication 21, **caractérisé en ce que** ledit troisième fil est un fil multifilaments en acide polyglycolique.

24. Tissu selon la revendication 21, **caractérisé en ce que** ledit troisième fil est un fil multifilaments en acide polylactique et glycolique.

25. Tissu selon l'une quelconque des revendications précédentes, **caractérisé en ce que** ladite face dense comprend au moins une nappe de premiers fils, tricotés serrés et déterminant une face unie mais perméable.

26. Tissu selon l'une quelconque des revendications 1 à 25, **caractérisé en ce que** ladite face poreuse comprend au moins deux nappes de deuxièmes fils, tricotés, déterminant des ouvertures de forme hexagonale.

27. Tissu selon la revendication 26, **caractérisé en ce que** ladite entretoise comprend au moins une nappe de troisièmes fils, s'étendant sensiblement perpendiculairement depuis la face poreuse vers la face dense, lesdits troisièmes fils étant distribués, au niveau de ladite face poreuse, selon les bords périphériques desdites ouvertures hexagonales.

28. Tissu selon la revendication précédente, **caractérisé en ce que** lesdits troisièmes fils forment des canaux transversaux, sensiblement parallèles les uns aux autres, dont la section interne est exempte de fils, lesdits canaux débouchant de part et d'autre du tissu, sur la face poreuse selon lesdites ouvertures hexagonales, et sur la face dense, respectivement.

29. Tissu selon la revendication précédente, **caractérisé en ce que** le diametre moyen des canaux transversaux est égal ou supérieur à 0,3 mm, de préférence allant de 0,7 à 3 mm, et de préférence encore allant de 1,3 à 1,7 mm.

30. Tissu selon la revendication précédente, **caractérisé en ce que** les canaux transversaux présentent une longueur, correspondant à l'épaisseur dudit tissu, allant de 0.5 à 5 mm, de préférence allant de 1,5 à 3 mm.

31. Tissu selon l'une quelconque des revendications 28 à 30, **caractérisé en ce que** l'entretoise détermine pour chaque canal une paroi interne poreuse d'interconnexion avec les canaux voisins, ladite paroi interne poreuse définissant des interstices de passage entre canaux.

32. Tissu selon la revendication précédente, **caractérisé en ce que** lesdits interstices de passage entre canaux ont une largeur allant de 100 à 300 microns

33. Prothèse pour renforcer, protéger ou soutenir une paroi tissulaire **caractérisée en ce qu'**elle est obtenue par découpage à partir d'un tissu prothétique selon l'une quelconque des revendications 1 à 32.

34. Procédé de préparation d'un tissu prothétique tridimensionnel comprer ant une première et une deuxième faces, ladite première face et ladite deuxième face étant opposées et séparées l'une de l'autre par l'épaisseur dudit tissu, ladite première face étant poreuse, ladite deuxième face étant dense et résorbable, comprenant une étape de fabrication d'un tricot tridimensionnel sur un métier chaîne ou Rachel selon au moins une nappe de fils définissant la face poreuse, au moins une nappe de fils définissant l'épaisseur dudit tissu, et au moins une nappe de fil définissant ladite face dense, **caractérisé en ce que** ladite nappe définissant ladite face dense est obtenue à l'aide d'une barre à passettes enfilée pleine avec au moins un premier fil, résorbable.

35. Procédé de préparation d'un tissu prothétique tridimensionnel comprenant une première et une deuxième faces, ladite première face et ladite deuxiéme face étant opposées et séparées l'une de l'autre par l'épaisseur dudit tissu, ladite première face étant poreuse, ladite deuxième face étant dense et résorbable, comprenant une étape de fabrication d'un tricot tridimensionnel sur un métier chaîne ou Rachel selon au moins une nappe de fils définissant la face poreuse, au moins une nappe de fils définissant l'épaisseur dudit tissu, et au moins une nappe de fil définissant ladite face dense, **caractérisé en ce que**,
- dans une première étape, ladite nappe définissant ladite face dense est obtenue à l'aide d'une barre à passettes enfilée pleine avec au moins, un premier fil en cellulose régénérée, et
- dans une deuxième étape, ledit tissu est soumis à une étape d'oxydation.

36. Procédé selon la revendication précédente, **caractérisé en ce que** ledit tissu est soumis à une oxydation par l'acide périodique ou par le metapériodate de sodium, puis rincé dans une solution aqueuse d'acétone ou d'alcool, et lavé à l'acétone ou à l'alcool purs avant séchage.

37. Procédé selon la revendication 35, **caractérisé en ce que** ledit tissu es soumis à une oxydation par le dioxyde d'azote gazeux à une température allant de 20 à 50°C, notamment de 25 à 40°C.

38. Procédé selon la revendication 35 ou 37, **caractérisé en ce que** ledit tissu est soumis à une oxydation par le dioxyde d'azote gazeux pendant une durée allant de 2 à 48 heures, notamment de 3 à 8 heures.

39. Procédé selon la revendication 37 ou 38, **caractérisé en ce que** la concentration en dioxyde d'azote est inférieure à 14g/L, notamment va de 6 à 12g/L

40. Procédé selon l'une quelconque des revendications 37 à 39, **caractérisé en ce que** le rapport massique NO₂/cellulose est supérieur à 0,9.

41. Procédé selon la revendication 35, **caractérisé en ce que** ledit tissu est soumis à une oxydation par le dioxyde d'azote gazeux en présence d'air cu d'oxygène.

42. Procédé selon la revendication 35, **caractérisé en ce que** ledit tissu, après une oxydation par le dioxyde d'azote gazeux, est soumis à une étape de lavage par un gaz inerte comme le CO₂ ou le N₂ pour éliminer l'excès en NO₂, suivie d'un lavage par un alcool volatil.

43. Procédé selon la revendication 35, **caractérisé en ce que** ledit tissu est soumis à une oxydation par le dioxyde d'azote dans un solvant non aqueux approprié puis lavé par une solution aqueuse d'isopropanol.

44. Procédé selon la revendication 42 ou 43, **caractérisé en ce qu'**un agent plastifiant, tel que la glycérine ou le polyéthylène glycol est ajouté à l'étape de lavage.

45. Procédé selon l'une quelconque des revendications 34 à 44, **caractérisé en ce que** lesdits premiers fils de la nappe définissant la face dense sont tricotés selon le barème 1112/1110//.

46. Procédé selon l'une quelconque des revendications 34 à 44, **caractérisé en ce que** lesdits premiers fils de la nappe définissant la face dense sont tricotés selon le barème 2223/1110//.

47. Procédé selon la revendication 35, **caractérisé en ce que** le tissu es stabilisé par passage au four à une température allant de 80 à 150°C avant oxydation.

## Claims

1. Three-dimensional prosthetic fabric comprising a first face and a second face, the said first face and the said second face being opposite each other and separated by the thickness of the said fabric, the said first face being porous, **characterized in that** the said second face is dense and is made of at least one first resorbable yarn, the said first porous face is made of at least one second resorbable or non-resorbable yarn, consisting of monofilaments and/or multifilaments of a biocompatible polymer material, the said first face and the said second face are linked together via a rib made of at least one third resorbable or non-resorbable yarn, consisting of monofilaments and/or multifilaments of a biocompatible polymer material.

2. Fabric according to the preceding claim, **characterized in that** the said first resorbable yarn consists of monofilaments and/or multifilaments of a biocompatible polymer material chosen from polyesters, polycaprolactones, polydioxanones, polyamides, polyethers and polysaccharides, and mixtures thereof.

3. Fabric according to the preceding claim, **characterized in that** the polyesters are chosen from polyhydroxy acids, preferably glycolic acid polymers, lactic acid polymers and hydroxybutyric acid polymers, and mixtures thereof.

4. Fabric according to Claim 2, **characterized in that** the polysaccharides are chosen from hyaluronic acid, alginic acid, polyglucuronic acid, chitosan, starch and soluble cellulose derivatives, and mixtures thereof.

5. Fabric according to the preceding claim, **characterized in that** said polysaccharide is crosslinked.

6. Fabric according to claim 4, **characterized in that** the soluble cellulose derivatives are chosen from cellulose ethers, for instance carboxymethylcellulose, and oxidized celluloses, and mixtures thereof.

7. Fabric according to the preceding claim, **characterized in that** the oxidized celluloses are chosen from oxidized cellulose in which the C₆ primary alcohol is partially or totally oxidized to a carboxylic acid, for example, to give polyglucuronic acid, cellulose oxidized in the form of polyaldehydes with periodic acid, and cellulose of "viscose" type, manufactured from a solubilized and then regenerated and oxidized cellulose pulp, and mixtures thereof.

8. Fabric according to Claim 7, **characterized in that** the said first resorbable yarn is a regenerated and oxidized cellulose multifilament yarn.

9. Fabric according to Claims 3 and 7, **characterized in that** the said first resorbable yarn is a composite multifilament yarn of polyglycolic acid and of oxidized cellulose in polyglucuronic acid form.

10. Fabric according to Claim 4, **characterized in that** the said first resorbable yarn is obtained by mixing a negatively charged polysaccharide, chosen from alginic acid, hyaluronic acid, polyglucuronic acid and mixtures thereof, and a positively charged polysaccharide, for instance chitosan.

11. Fabric according to any one of the preceding claims, **characterized in that**, since the said first resorbable yarn is a multifilament yarn composed of multiple filaments defining between themselves interstitial spaces, the said multifilament yarn is impregnated, in the said interstitial spaces, with a polysaccharide chosen from hyaluronic acid, alginic acid, polyglucuronic acid, chitosan, starch and soluble cellulose derivatives, and mixtures thereof.

12. Fabric according to the preceding claim, **characterized in that** the said first resorbable yarn is a multifilament yarn of polyglycolic acid impregnated with polyglucuronic acid.

13. Fabric according to Claim 11, **characterized in that** the said first resorbable yarn is a multifilament yarn of polyglycolic acid impregnated with a mixture of polyglucuronic acid and chitosan.

14. Fabric according to claim 11, **characterized in that** the said first resorbable yarn is a multifilament yarn of polyglycolic acid impregnated with a mixture of hyaluronic acid and chitosan.

15. Fabric according to Claim 11, **characterized in that** the said first resorbable yarn is a multifilament yarn of polyglycolic acid impregnated with hyaluronate crosslinked with 1,4-butanediol diglycidyl ether.

16. Fabric according to claim 11, **characterized in that** the said first resorbable yarn is a multifilament yarn of oxidized cellulose impregnated with chitosan.

17. Fabric according to anyone of the preceding claims, **characterized in that** the said second yarn is non-resorbable.

18. Fabric according to Claim 17, **characterized in that** the said second yarn consists of monofilaments and/or multifilaments of a biocompatible polymer material chosen from polypropylene, polyethylene terephthalate, polytetrafluoroethylene, polyamide, polyvinyldifluorene, and mixtures thereof.

19. Fabric according to anyone of the preceding claims, **characterized in that** the said third yarn is resorbable.

20. Fabric according to the preceding claim, **characterized in that** the said third yarn consists of monofilaments and/or multifilaments of a biocompatible polymer material chosen from polyesters, polycaprolactones, polydioxanones, polyalkanoates, polyamides, polyphosphazenes, polyacetals, polyurethanes, polyorthoesters, polycarbonates and polyanhydrides, and mixtures thereof.

21. Fabric according to the preceding claim, **characterized in that** the polyesters are chosen from polyhydroxy acids, preferably glycolic acid polymers, lactic acid polymers and hydroxybutyric acid polymers, and mixtures thereof.

22. Fabric according to the preceding claim, **characterized in that** the said third yarn is a polylactic acid multifilament yarn.

23. Fabric according to Claim 21, **characterized in that** the said third yarn is a polyglycolic acid multifilament yarn.

24. Fabric according to Claim 21, **characterized in that** the said third yarn is a polylactic and glycolic acid multifilament yarn.

25. Fabric according to any one of the preceding claims, **characterized in that** the said dense face comprises at least one lap of first close-knitted yarns which determines a unified but permeable face.

26. Fabric according to any one Claims 1 to 25, **characterized in that** the said porous face comprises at least two laps of knitted second yarns, determining hexagonal-shaped apertures.

27. Fabric according to Claim 26, **characterized in that** the said rib comprises at least one lap of third yarns, extending substantially perpendicularly from the porous face to the dense face, the said third yarns being distributed, on the said porous face, along the peripheral edges of the said hexagonal apertures.

28. Fabric according to the preceding claim, **characterized in that** the said third yarns form substantially parallel transverse channels, the internal cross section of which is free of yarns, the said channels emerging on either side of the fabric, on the porous face according to the said hexagonal apertures, and on the dense face, respectively.

29. Fabric according to the preceding claim, **characterized in that** the mean diameter of the transverse channels is greater than or equal to 0.3 mm, preferably ranging from 0.7 to 3 mm and more preferably ranging from 1.3 to 1.7 mm.

30. Fabric according to the preceding claim, **characterized in that** the transverse channels have a length, corresponding to the thickness of the said fabric, ranging from 0.5 to 5 mm and preferably ranging from 1.5 to 3 mm.

31. Fabric according to any one of Claims 28 to 30, **characterized in that** the rib determines for each channel a porous inner wall interconnecting with the neighbouring channels, the said porous inner wall defining interstices for passing between channels.

32. Fabric according to the preceding claim, **characterized in that** the said interstices for passing between channels have a width ranging from 100 to 300 microns.

33. Prosthesis for reinforcing, protecting or supporting a tissue wall, **characterized in that** it is obtained by cutting out from a prosthetic fabric according to any one of Claims 1 to 32.

34. Process for preparing a three-dimensional prosthetic fabric comprising a first face and a second face, the said first face and the said second face being opposite each other and separated by the thickness of the said fabric, the said first face being porous, the said second face being dense and resorbable, comprising a step of manufacturing a three-dimensional knitted fabric on a warp knitting machine or a Raschel knitting machine in at least one lap of yarns defining the porous face, at least one lap of yarns defining the thickness of the said fabric, and at least one lap of yarn defining the said dense face, **characterized in that** the said lap defining the said dense face is obtained using a full-threaded guide bar with at least one first resorbable yarn.

35. Process for preparing a three-dimensional prosthetic fabric comprising a first face and a second face, the said first face and the said second face being opposite each other and separated by the thickness of the said fabric, the said first face being porous, the said second face being dense and resorbable, comprising a step of manufacturing a three-dimensional knitted fabric on a warp knitting machine or Raschel knitting machine in at least one lap of yarns defining the porous face, at least one lap of yarns defining the thickness of the said fabric, and at least one lap of yarn defining the said dense face, **characterized in that**:
- in a first step, the said lap defining the said dense face is obtained using a full-threaded guide bar with at least one first regenerated cellulose yarn, and
- in a second step, the said fabric is subjected to an oxidation step.

36. Process according to the preceding claim, **characterized in that** the said fabric is subjected to oxidation with sodium periodate or with periodic acid and is then rinsed in an aqueous acetone or alcohol solution, and washed with pure acetone or alcohol before drying.

37. Process according to Claim 35, **characterized in that** the said fabric is subjected to oxidation with gaseous nitrogen dioxide at a temperature ranging from 20 to 50°C, in particular from 25 to 40°C.

38. Process according to Claim 35 or 37, **characterized in that** the said fabric is subjected to oxidation with gaseous nitrogen dioxide for a time ranging from 2 to 48 hours, in particular from 3 to 8 hours.

39. Process according to Claim 37 or 38, **characterized in that** the nitrogen dioxide concentration is less than 14 g/L, in particular ranges from 6 to 12 g/L.

40. Process according to any of Claims 37 to 39, **characterized in that** the weight ratio NO₂/cellulose is greater than 0.9.

41. Process according to Claim 35, **characterized in that** the said fabric is subjected to oxidation with gaseous nitrogen dioxide in the presence of air or oxygen.

42. Process according to Claim 35, **characterized in that** after oxidation with nitrogen dioxide, said fabric is subjected to a washing step with an inert gas like CO₂ or N₂ in order to remove the excess of NO₂, followed by a washing with a volatile alcohol.

43. Process according to Claim 35, **characterized in that** the said fabric is subjected to oxidation with nitrogen dioxide in a suitable non-aqueous solvent and is then washed with an aqueous isopropanol solution.

44. Process according to Claim 42 or 43, **characterized in that** a plasticizer, such as glycerol or polyethylene glycol, is added to the washing step.

45. Process according to any one of Claims 34 to 44, **characterized in that** the said first yarns of the lap defining the dense face are knitted according to the scale 1112/1110//.

46. Process according to any one of Claims 34 to 44, **characterized in that** the said first yarns of the lap defining the dense face are knitted according to the scale 2223/1110//.

47. Process according to Claim 35, **characterized in that**, before oxidation, the fabric is stabilized by baking at a temperature ranging from 80 to 150°C.

## Patentansprüche

1. Prothetisches dreidimensionales Gewebe, das eine erste und eine zweite Seite aufweist, wobei die erste Seite und die zweite Seite einander gegenüberliegen und durch die Dicke des Gewebes voneinander getrennt sind, wobei die erste Seite porös ist, **dadurch gekennzeichnet, dass** die zweite Seite dicht ist und aus zumindest einer resorbierbaren ersten Faser besteht, dass die poröse erste Seite aus zumindest einer resorbierbaren oder nicht resorbierbaren zweiten Faser besteht, die aus Monofilamenten und/oder Multifilamenten aus einem biokompatiblen Polymermaterial besteht, und dass die erste Seite und die zweite Seite durch ein Zwischenstück miteinander verbunden sind, das aus zumindest einer resorbierbaren oder nicht resorbierbaren dritten Faser besteht, die aus Monofilamenten und/oder Multifilamenten aus einem biokompatiblen Polymermaterial besteht.

2. Gewebe nach dem vorhergehenden Anspruch, **dadurch gekennzeichnet, dass** die resorbierbare erste Faser aus Monofilamenten und/oder Multifilamenten aus einem biokompatiblen Polymermaterial besteht, das ausgewählt ist aus den Polyestern, den Polycaprolactonen, den Polydioxanonen, den Polyamiden, den Polyethern, den Polysacchariden und deren Mischungen.

3. Gewebe nach dem vorhergehenden Anspruch, **dadurch gekennzeichnet, dass** die Polyester ausgewählt sind aus den Polyhydroxysäuren, vorzugsweise den Polymeren von Glykolsäure, den Polymeren von Milchsäure, den Polymeren von Hydroxybuttersäure und deren Mischungen.

4. Gewebe nach Anspruch 2, **dadurch gekennzeichnet, dass** die Polysaccharide ausgewählt sind aus Hyaluronsäure, Alginsäure, Polyglucuronsäure, Chitosan, Amidon, den löslichen Zellulosederivaten, deren Salzen und deren Mischungen.

5. Gewebe nach dem vorhergehenden Anspruch, **dadurch gekennzeichnet, dass** das Polysaccharid vernetzt ist.

6. Gewebe nach Anspruch 4, **dadurch gekennzeichnet, dass** die löslichen Zellulosederivate ausgewählt sind aus den Zelluloseethern, wie Carboxymethylzellulose, den oxidierten Zellulosen und deren Mischungen.

7. Gewebe nach dem vorhergehenden Anspruch, **dadurch gekennzeichnet, dass** die oxidierten Zellulosen ausgewählt sind aus einer oxidierten Zellulose, in der der primäre Alkohol in C₆ teilweise oder vollständig zur Carbonsäure oxidiert ist, z.B. um Polyglucuronsäure zu ergeben, einer durch Periodsäure oxidierten Zellulose in Form von Polyaldehyden, einer Zellulose vom Typ "Viskose", die ausgehend von einer solubilisierten Zellulosepaste hergestellt und anschließend regeneriert und oxidiert wurde, und deren Mischungen.

8. Gewebe nach Anspruch 7, **dadurch gekennzeichnet, dass** die resorbierbare erste Faser eine Multifilamentfaser aus regenerierter und oxidierter Zellulose ist.

9. Gewebe nach Anspruch 3 und 7, **dadurch gekennzeichnet, dass** die resorbierbare erste Faser eine Multifilament-Kompositfaser aus Polyglykolsäure und oxidierter Zellulose in Form von Polyglucuronsäure ist.

10. Gewebe nach Anspruch 4, **dadurch gekennzeichnet, dass** die resorbierbare erste Faser durch Vermischen eines Polysaccharids mit negativer Ladung, das ausgewählt ist aus Alginsäure, Hyaluronsäure, Polyglucuronsäure und deren Mischungen und eines Polysaccharids mit positiver Ladung, wie Chitosan, erhalten wird.

11. Gewebe nach einem der vorhergehenden Ansprüche, **dadurch gekennzeichnet, dass** die resorbierbare erste Faser eine Multifilamentfaser ist, die aus einer Vielzahl von Filamenten besteht, die zwischeneinander Zwischenräume bilden, und dass die Multifilamentfaser auf Höhe der Zwischenräume mit einem Polysaccharid imprägniert ist, das ausgewählt ist aus Hyaluronsäure, Alginsäure, Polyglucuronsäure, Chitosan, Amidon, den löslichen Zellulosederivaten und deren Mischungen.

12. Gewebe nach dem vorhergehenden Anspruch, **dadurch gekennzeichnet, dass** die resorbierbare erste Faser eine Multifilamentfaser aus Polyglykolsäure ist, die mit Polyglucuronsäure imprägniert ist.

13. Gewebe nach Anspruch 11, **dadurch gekennzeichnet, dass** die resorbierbare erste Faser eine Multifilamentfaser aus Polyglykolsäure ist, die mit einer Mischung aus Polyglucuronsäure und Chitosan imprägniert ist.

14. Gewebe nach Anspruch 11, **dadurch gekennzeichnet, dass** die resorbierbare erste Faser eine Multifilamentfaser aus Polyglykolsäure ist, die mit einer Mischung aus Hyaluronsäure und Chitosan imprägniert ist.

15. Gewebe nach Anspruch 11, **dadurch gekennzeichnet, dass** die resorbierbare erste Faser eine Multifilamentfaser aus Polyglykolsäure ist, die mit Hyaluronsäure imprägniert ist, die mit 1,4-Butandioldiglycidylether vernetzt ist.

16. Gewebe nach Anspruch 11, **dadurch gekennzeichnet, dass** die resorbierbare erste Faser eine Multifilamentfaser aus oxidierter Zellulose ist, die mit Chitosan imprägniert ist.

17. Gewebe nach einem der vorhergehenden Ansprüche, **dadurch gekennzeichnet, dass** die zweite Faser nicht resorbierbar ist.

18. Gewebe nach Anspruch 17, **dadurch gekennzeichnet, dass** die zweite Faser aus Monofilamenten und/oder Multifilamenten aus einem biokompatiblen Polymermaterial besteht, das ausgewählt ist aus Polypropylen, Polyethylenterephthalat, Polytetrafluorethylen, Polyamid, Polyvinyldifluoren und deren Mischungen.

19. Gewebe nach einem der vorhergehenden Ansprüche, **dadurch gekennzeichnet, dass** die dritte Faser resorbierbar ist.

20. Gewebe nach dem vorhergehenden Anspruch, **dadurch gekennzeichnet, dass** die dritte Faser aus Monofilamenten und/oder Multifilamenten aus einem biokompatiblen Polymermaterial besteht, das ausgewählt ist aus den Polyestern, den Polycaprolactonen, den Polydioxanonen, den Polyalkanoaten, den Polyamiden, den Polyphosphazenen, den Polyacetalen, den Polyurethanen, den Polyortlioesteru, den Polycarbonaten, den Polyanhydriden und deren Mischungen.

21. Gewebe nach dem vorhergehenden Anspruch, **dadurch gekennzeichnet, dass** die Polyester ausgewählt sind aus den Polyhydroxysäuren, vorzugsweise aus den Polymeren von Glykolsäure, den Polymeren von Milchsäure, den Polymeren von Hydroxybuttersäure und deren Mischungen.

22. Gewebe nach dem vorhergehenden Anspruch, **dadurch gekennzeichnet, dass** die dritte Faser eine Multifilamentfaser aus Polymilchsäure ist.

23. Gewebe nach Anspruch 21, **dadurch gekennzeichnet, dass** die dritte Faser eine Multifilamentfaser aus Polyglykolsäure ist.

24. Gewebe nach Anspruch 21, **dadurch gekennzeichnet, dass** die dritte Faser eine Multifilamentfaser aus Polymilchsäure und Glykolsäure ist.

25. Gewebe nach einem der vorhergehenden Ansprüche, **dadurch gekennzeichnet, dass** die dichte Seite zumindest ein Faservlies aus ersten Fasern aufweist, die eng gestrickt sind und eine einheitliche, aber permeable Seite bilden.

26. Gewebe nach einem der Ansprüche 1 bis 25, **dadurch gekennzeichnet, dass** die poröse Seite zumindest zwei Faservliese aus zweiten Fasern aufweist, die gestrickt sind und die Öffnungen mit hexagonaler Form bilden.

27. Gewebe nach Anspruch 26, **dadurch gekennzeichnet, dass** das Zwischenstück zumindest ein Faservlies aus dritten Fasern aufweist, die sich ungefähr im rechten Winkel von der porösen Seite zu der dichten Seite erstrecken, wobei die dritten Fasern auf Höhe der porösen Seite entlang der äußeren Ränder der hexagonalen Öffnungen verteilt sind.

28. Gewebe nach dem vorhergehenden Anspruch, **dadurch gekennzeichnet, dass** die dritten Fasern Querkanäle bilden, die ungefähr parallel zueinander verlaufen, und deren interner Querschnitt keine Fasern aufweist, wobei sich die Kanäle auf beiden Seiten des Gewebes entlang den hexagonalen Öffnungen zu der porösen Seite bzw. zu der dichten Seite hin öffnen.

29. Gewebe nach dem vorhergehenden Anspruch, **dadurch gekennzeichnet, dass** der mittlere Durchmesser der Querkanäle größer oder gleich 0,3 mm ist, und vorzugsweise bei 0,7 bis 3 mm, und noch bevorzugter bei 1,3 bis 1,7 mm liegt.

30. Gewebe nach dem vorhergehenden Anspruch, **dadurch gekennzeichnet, dass** die Querkanäle eine Länge aufweisen, die der Dicke des Gewebes entspricht und die bei 0,5 bis 5 mm, vorzugsweise bei 1,5 bis 3 mm liegt.

31. Gewebe nach einem der Ansprüche 28 bis 30, **dadurch gekennzeichnet, dass** das Zwischenstück für jeden Kanal eine interne poröse Wand zur Verbindung mit den benachbarten Kanälen bildet, wobei die interne poröse Wand Durchgangszwischenräume zwischen den Kanälen bildet.

32. Gewebe nach dem vorhergehenden Anspruch, **dadurch gekennzeichnet, dass** die Durchgangszwischenräume zwischen den Kanälen eine Breite von 100 bis 300 Mikron aufweisen.

33. Prothese zum Verstärken, Schützen oder Abstützen einer Gewebewand, **dadurch gekennzeichnet, dass** diese durch Zuschneiden eines prothetischen Gewebes nach einem der Ansprüche 1 bis 32 erhalten wurde.

34. Verfahren zur Herstellung eines dreidimensionalen prothetischen Gewebes, das eine erste und eine zweite Seite aufweist, wobei die erste Seite und die zweite Seite einander gegenüberliegen und durch die Dicke des Gewebes voneinander getrennt sind, wobei die erste Seite porös ist, und wobei die zweite Seite dicht und resorbierbar ist, das einen Schritt der Herstellung eines dreidimensionalen Strickgewebes auf einer Kettenwirkmaschine oder einer Rachel-Maschine nach zumindest einem Faservlies aus Fasern, das die poröse Seite bildet, zumindest einem Faservlies aus Fasern, das die Dicke des Gewebes bildet, und zumindest einem Faservlies aus Fasern, das die dichte Seite bildet, aufweist, **dadurch gekennzeichnet, dass** das Faservlies, das die dichte Seite bildet, mithilfe eines Legebarrens erhalten wird, der mit zumindest einer resorbierbaren ersten Faser vollständig umwickelt ist.

35. Verfahren zur Herstellung eines dreidimensionalen prothetischen Gewebes, das eine erste und eine zweite Seite aufweist, wobei die erste Seite und die zweite Seite einander gegenüberliegen und durch die Dicke des Gewebes voneinander getrennt sind, wobei die erste Seite porös ist und wobei die zweite Seite dicht und resorbierbar ist, das einen Schritt der Herstellung eines dreidimensionalen Strickgewebes auf einer Kettenwirkmaschine oder einer Rachel-Maschine nach zumindest einem Faservlies aus Fasern, das die poröse Seite bildet, zumindest einem Faservlies aus Fasern, das die Dicke des Gewebes bildet, und zumindest einem Faservlies aus Fasern, das die dichte Seite bildet, aufweist, **dadurch gekennzeichnet,**
- **dass** in einem ersten Schritt das Faservlies, das die dichte Seite bildet, mithilfe eines Legebarrens erhalten wird, der mit zumindest einer ersten Faser aus regenerierter Zellulose vollständig umwickelt ist, und,
- **dass** in einem zweiten Schritt das Gewebe einem Schritt der Oxidation unterzogen wird.

36. Verfahren nach dem vorhergehenden Anspruch, **dadurch gekennzeichnet, dass** das Gewebe einer Oxidation mit Periodsäure oder mit Natriummetaiodad unterzogen wird und dann in einer wässrigen Lösung aus Aceton oder Alkohol gespült und vor dem Trocknen mit reinem Aceton oder reinem Alkohol gewaschen wird.

37. Verfahren nach Anspruch 35, **dadurch gekennzeichnet, dass** das Gewebe bei einer Temperatur von 20 bis 50°C, insbesondere von 25 bis 40°C, einer Oxidation mit gasförmigem Stickstoffdioxid unterzogen wird.

38. Verfahren nach Anspruch 35 oder 37, **dadurch gekennzeichnet, dass** das Gewebe für eine Dauer von 2 bis 48 Stunden, insbesondere von 3 bis 8 Stunden einer Oxidation mit gasförmigem Stickstoffdioxid unterzogen wird.

39. Verfahren nach Anspruch 37 oder 38, **dadurch gekennzeichnet, dass** die Konzentration an Stickstoffdioxid unter 14 g/l, insbesondere bei 6 bis 12 g/l liegt.

40. Verfahren nach einem der Ansprüche 37 bis 39, **dadurch gekennzeichnet, dass** das Massenverhältnis von NO₂/Zellulose größer als 0,9 ist.

41. Verfahren nach Anspruch 35, **dadurch gekennzeichnet, dass** das Gewebe in Gegenwart von Luft oder Sauerstoff einer Oxidation mit gasförmigem Stickstoffdioxid unterzogen wird.

42. Verfahren nach Anspruch 35, **dadurch gekennzeichnet, dass** das Gewebe nach einer Oxidation mit gasförmigem Stickstoffdioxid einem Schritt des Waschens mit einem inerten Gas wie CO₂ oder N₂ unterzogen wird, um einen Überschuss an NO₂ zu entfernen, gefolgt von einem Waschen mit einem flüchtigen Alkohol.

43. Verfahren nach Anspruch 35, **dadurch gekennzeichnet, dass** das Gewebe in einem geeigneten, nicht wässrigen Lösemittel einer Oxidation mit Stickstoffdioxid unterzogen wird, gefolgt von einem Waschen mit einer wässrigen Lösung von Isopropanol.

44. Verfahren nach Anspruch 42 oder 43, **dadurch gekennzeichnet, dass** ein plastifizierendes Mittel wie bspw. Glyzerin oder Polyethylenglykol im Schritt des Waschens zugegeben wird.

45. Verfahren nach einem der Ansprüche 34 bis 44, **dadurch gekennzeichnet, dass** die ersten Fasern des Faservlieses, das die dichte Seite bildet, nach dem Muster 1112/1110// gestrickt werden.

46. Verfahren nach einem der Ansprüche 34 bis 44, **dadurch gekennzeichnet, dass** die ersten Fasern des Faservlieses, das die dichte Seite bildet, nach dem Muster 2223/1110// gestrickt werden.

47. Verfahren nach Anspruch 35, **dadurch gekennzeichnet, dass** das Gewebe vor der Oxidation durch einen Durchgang durch einen Ofen bei einer Temperatur von 80 bis 150°C stabilisiert wird.
